**Europäisches Patentamt**

**European Patent Office**

**Office européen des brevets**

(19)

(11) Veröffentlichungsnummer: **0 286 914 B1**

# (12) EUROPÄISCHE PATENTSCHRIFT

(45) Veröffentlichungstag der Patentschrift:
20.03.91 Patentblatt 91/12

(51) Int. Cl.$^5$: **C07C 309/03**, C07C 303/02

(21) Anmeldenummer: **88105147.8**

(22) Anmeldetag: **30.03.88**

(54) Verfahren zur Herstellung von Trifluormethansulfonsäure.

(30) Priorität: **11.04.87 DE 3712318**

(43) Veröffentlichungstag der Anmeldung:
**19.10.88 Patentblatt 88/42**

(45) Bekanntmachung des Hinweises auf die
Patenterteilung:
**20.03.91 Patentblatt 91/12**

(84) Benannte Vertragsstaaten:
**CH DE ES FR GB IT LI**

(56) Entgegenhaltungen:
**Keine Entgegenhaltungen**

(73) Patentinhaber: **BAYER AG**
**W-5090 Leverkusen 1 Bayerwerk (DE)**

(72) Erfinder: **Bielefeldt, Dietmar, Dr.**
**Beuthener Strasse 13**
**W-4030 Ratingen 6 (DE)**
Erfinder: **Marhold, Albrecht, Dr.**
**Carl-Duisberg-Strasse 329**
**W-5090 Leverkusen 1 (DE)**

EP 0 286 914 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein Verfahren zur Herstellung von Trifluormethansulfonsäure aus Bis-(trifluormethyl)-disulfan.

Trifluormethansulfonsäure ist eine universell verwendbare Chemikalie, die beispielsweise für die Herstellung von Pflanzenschutzmitteln benötigt wird (siehe JP-OS 85-243 061) und als Acylierungskatalysator eingesetzt werden kann (siehe Chem. Reviews, 77, 69-92 (1977)).

Gemäß dem einzigen bekannten Verfahren, das ausgehend von Disulfiden Trifluormethansulfonsäure liefert, wird Di-(dichlorfluormethyl)-disulfid mit Fluorwasserstoff und Sauerstoff in der Gasphase bei 340 bis 410°C in Gegenwart von Deacon-Katalysatoren, das sind im allgemeinen Oxide oder Halogenide mehrwertiger Metalle mit variablen Wertigkeitszuständen, umgesetzt (siehe DE-OS 26 58 139, insbesondere Beispiel 195). Nachteilig sind bei diesem Verfahren die hohen anzuwendenden Temperaturen, die dabei mit Fluorwasserstoff und Sauerstoff auftretenden Korrosionsprobleme, das Anfallen von Trifluormethansulfonsäure im Gemisch mit Difluormonochlor- und Monofluordichlormethansulfonsäure, das Fehlen von Angaben über die absoluten und relativen Mengen, in denen Trifluormethansulfonsäure erhalten wurde und das Fehlen von Angaben, ob und gegebenenfalls wie man aus dem Reaktionsgemisch Trifluormethansulfonsäure isolieren kann.

Es wurde nun ein Verfahren zur Herstellung von Trifluormethansulfonsäure gefunden, das dadurch gekennzeichnet ist, daß man Bis-(trifluormethyl)-disulfan in flüssiger Phase bei – 20 bis + 100°C und in saurem Milieu mit einem sauerstoffhaltigen Oxidationsmittel behandelt.

Das für das erfindungsgemäße Verfahren benötigte Ausgangsprodukt Bis-(trifluormethyl)-disulfan ist bekannt und auf einfache Weise zugänglich. Man erhält es beispielsweise als bisher unerwünschtes Nebenprodukt bei der Herstellung von CF$_3$SCl (siehe W. Tullock und D.D. Coffmann, J. org. Chem., 25, 2016 (1960)), durch Fluorierung von Trichlormethylsulfenylchlorid mit Natriumfluorid (siehe US-PS 2 884 453) oder durch Fluorierung von Bis-(trichlormethyldisulfid) mit Fluorwasserstoff (siehe DE-OS 1 232 954 und Synthesis 6, 310 (1972)).

Die erfindungsgemäß erforderliche flüssige Phase und das erfindungsgemäß erforderliche saure Milieu können beispielsweise verifiziert werden, indem man in Gegenwart einer starken Säure arbeitet, die bei den angewendeten Reaktionsbedingungen flüssig ist. Vorsugsweise ist diese Säure wasserfrei oder wasserarm. Geeignet sind beispielsweise Schwefelsäure in Konzentrationen von 70 bis 100 Gew.-%, Oleum mit Gehalten an freiem SO$_3$ von 0 bis

100 Gew.-%, Phosphorsäure in konzentrationen von 85 bis 100 Gew.-% und Essigsäure in Konzentrationen von 90 bis 100 Gew.-%, sowie Fluor enthaltende organische und anorganische Säuren wie Fluoressigsäuren, insbesondere Trifluoressigsäure, Perfluoralkansulfonsäuren und Fluorsulfonsäure. Es können auch Gemische von mehreren Säuren eingesetzt werden.

Die Menge der Säure ist nicht kritisch. Beispielsweise kann man pro Gewichtsteil Bis-(trifluormethyl)-disulfan 5 bis 20 Gewichtsteile Säure einsetzen.

Die erfindungsgemäß einzusetzenden sauerstoffhaltigen Oxidationsmittel können von verschiedener Art sein. Beispielsweise kommen hierfür Stoffe in Frage, die peroxidischen Sauerstoff enthalten, z.B. Wasserstoffperoxid, anorganische Persäuren, wie Caro'sche Säure (H$_2$SO$_5$), oder Salze davon, organische Persäuren, wie aliphatische C$_1$- bis C$_4$-Percarbonsäuren und, gegebenenfalls substituierte, aromatische Percarbonsäuren, Peroxosalze, wie Peroxosulfate und Peroxoborate, sowie mit Fluor substituierte Schwefel-Peroxoverbindungen wie S$_2$O$_6$F$_2$. Als sauerstoffhaltige Oxidationsmittel kommen auch andere aktive Sauerstoff enthaltende Verbindungen in Frage, beispielsweise Ozon. Man kann auch sonstige Sauerstoff liefernde und/oder übertragende Verbindungen einsetzen, beispielsweise Salpetersäure, Nitrate, Oxide von Mettalen in höheren Wertigkeitsstufen, die entsprechenden Säuren und die entsprechenden Salze. Ebenso kann man auch molekularen Sauerstoff einsetzen, insbesondere zusammen mit Katalysatoren und/oder Aktivatoren.

Es kann vorteilhaft sein, das sauerstoffhaltige Oxidationsmittel gemeinsam mit einem Katalysator einzusetzen, z.B. Salpetersäure oder Königswasser und Vanadinpentoxid, oder molekularer Sauerstoff und Osmiumtetroxid, oder eine Peroxyverbindung und ein Schwermetallsalz.

Vorzugsweise setzt man als Oxidationsmittel ein: Wasserstoffperoxid, Peressig- oder Perpropionsäure, bevorzugt gelöst in Wasser oder einem inerten organischen Lösungsmittel, Perbenzoesäure, 3-Chlor-perbenzoesäure, Peroxosulfate, Peroxodisulfate und Caro'sche Säure.

Besonders bevorzugte Oxidationsmittel sind Wasserstoffperoxid, Caro'sche Säure und Peroxodisulfate.

Die eingestzten sauerstoffhaltigen Oxidationsmittel müssen nicht unbedingt in der eingesetzten Form mit Bis-(trifluormethyl)-disulfan reagieren, sondern können vor dieser Reaktion ganz oder teilweise in andere sauerstoffhaltige Oxidationsmittel umgewandelt werden. Beispielsweise kann aus Wasserstoffperoxid und Schwefelsäure Caro'sche Säure, aus Salpetersäure und Salzsäure ein Nitrosyl-Ionen enthaltendes Gemisch (= sogenanntes Königswasser) oder aus Wasserstoffperoxid und Essigsäure Peressigsäure entstehen.

Ebenso kann man das jeweils gewünschte sauerstoffhaltige Oxidationsmittel auch erst in situ entstehen lassen, z.B. Caro'sche Säure aus Wasserstoffperoxid und Schwefelsäure.

Die Menge des sauerstoffhaltigen Oxidationsmittels kann in weiten Grenzen variiert werden. Geeignet sind beispielsweise 2 bis 20 Äquivalente Oxidationsmittel pro Mol Bis-(trifluormethyl)-disulfan. Vorzugsweise beträgt diese Menge 5 bis 10 Äquivalente Oxidationsmittel pro Mol Bis-(trifluormethyl)-disulfan.

Vorzugsweise liegen die Temperaturen beim erfindungsgemäßen Verfahren im Bereich von 0 bis 60°C.

Das erfindungsgemäße Verfahren wird im allgemeinen bei Normaldruck durchgeführt. Will man jedoch bei Reaktionstemperaturen arbeiten, bei denen einzelne oder alle Komponenten des Reaktionsgemisches flüchtig sind, so arbeitet man zweckmäßigerweise unter erhöhtem Druck, beispielsweise in geschlossenen Gefäßen oder mit Aufdrücken eines Inertgases. Sofern die Flüchtigkeit des Reaktionsgemisches dies zuläßt kann man auch bei erniedrigten Drucken arbeiten. Aus praktischen Erwägungen wird vorteilhafterweise ein Druckbereich von 1 bis 6 bar eingehalten.

Das erfindungsgemäße Verfahren kann in Anwesenheit oder in Abwesenheit von Lösungsmitteln durchgeführt werden. Geeignete Lösungsmittel sind beispielsweise perfluorierte Ether und Sulfone, wie Tetramethylensulfon.

Lösungsmittel können als solche dem Reaktionsgemisch zugegeben werden, jedoch auch indem man eine oder mehrere für das erfindungsgemäße Verfahren benötigte Materialien in Form einer Lösung einsetzt.

Es ist vorteilhaft, die Menge von Wasser im Reaktionsgemisch zu minimieren, insbesondere bei Abwesenheit von Lösungsmitteln. Vorzugsweise versucht man während der Reaktion gebildetes Wasser zu entfernen oder unwirksam zu machen, beispielsweise beim Arbeiten in einem schwefelsauren Medium durch Zusatz von Schwefeltrioxid oder beim Arbeiten in Phosphorsäure durch Zusatz von Phosphorpentoxid.

Geeignete Reaktionszeiten für das erfindungsgemäße Verfahren sind beispielsweise solche im Bereich von 2 bis 72 Stunden.

Nach Durchführung des erfindungsgemäßen Verfahrens kann man Trifluormethansulfonsäure aus dem Reaktionsgemisch beispielsweise durch Destillation gewinnen. Man kann dabei so verfahren, daß man zunächst das Reaktionsgemisch entwässert und dann destilliert.

Mit dem erfindungsgemäßen Verfahren gelingt es Trifluormethansulfonsäure auf einfache Weise in guten Reinheiten und mit vorteilhaften Ausbeuten zu erhalten. Das erfindungsgemäße Verfahren zeichnet sich weiterhin dadurch aus, daß es bei relativ tiefen Temperaturen durchgeführt werden kann und, daß es hinsichtlich Korrosionsproblemen und Energiekosten vorteilhaft ist. Außerdem gestattet es die Herstellung von Trifluormethansulfonsäure in technischem Maßstab aus einem Ausgangsprodukt, für das bisher keine technische Verwendbarkeit bekannt ist.

Es ist ausgesprochen überraschend, daß mit dem erfindungsgemäßen Verfahren die aufgezeigten Vorteile erzielt werden können, denn in J. Chem. Sox. 4230 (1954) ist beschrieben, daß beim Versuch der Oxidation von Bis-(trifluormethyl)-disulfid mit konzentrierter Salpetersäure keine Reaktion auftrat und bei hohen Temperaturen nur unidentifizierte Zersetzungsprodukte gefunden werden.

Die nachfolgenden Beispiele erläutern das erfindungsgemäße Verfahren ohne es darauf zu beschränken.

Beispiele

Beispiel 1

Zu 540 g (2 Mol) Kaliumperoxodisulfat gelöst in 1000 ml Schwefelsäure der Dichte 1,832 (bei 20°C) wurden bei 20°C 220 g (1,1 Mol) $CF_3SSCF_3$ zugegeben. Das Reaktionsgemisch wurde bei 40°C 16 Stunden lang gerührt. Anschließend wurde das Reaktionsgemisch einer Destillation unterworfen. Zunächst wurden 86 g (0,43 Mol) nicht umgesetztes $CF_3SSCF_3$ zurückgewonnen. Anschließend wurden mit einem Siedepunkt von 80°C bei einem Druck von 0,5 mbar 44 g (19% der Theorie) Trifluormethansulfonsäure erhalten. Das erhaltene Produkt hatte einen Brechungsindex von $n_D^{20} = 1,341$ und ein $^{19}F$-NMR-Spektrum mit einem charakteristischen Signal bei δ = – 0.3 ppm (gemessen mit $CF_3CO_2H$ als externem Standard). Der Schmelzpunkt des aus der so erhaltenen Trifluormethansulfonsäure hergestellten N-Anilintrifluormethansulfonates betrug 268°C. Mit aus käuflicher Trifluormethansulfonsäure hergestelltem N-Anilintrifluormethansulfonat wurde keine Schmelzpunkterniedrigung gefunden.

Beispiel 2

Zu 405 g (2,0 Mol) $CF_3SSCF_3$, die in 2000 ml Schwefelsäure einer Dichte von 1,840 (bei 15°C) gelöst waren wurden zwischen 20 und 40°C 820 g (12,1 Mol) 50%iges wäßriges Wasserstoffperoxid hinzugefügt. Nach dem Ende der Zugabe wurde bei 20 bis 25°C über Nacht gerührt und anschließend 76 g (0,38 Mol) nicht umgesetztes $CF_3SSCF_3$ abdestilliert. Das zurückbleibende Produktgemisch wurde durch Zusatz von 4900 g 65 gew.-%igem Oleum wasserfrei gemacht. Danach wurde Trifluormethansulfonsäure abdestilliert und im Siedebereich von 20 bis 90°C bei 400 mbar 775 g rohes Produkt aufgefangen. Durch nochmalige Destillation über eine 30 cm-Vigreux-Kolonne wurde wasserfreie Trifluormethansulfonsäure erhalten. Die Ausbeute betrug 260 g (= 52% der Theorie). Der Siedepunkt der so isolierten Trifluormethansulfonsäure lag bei 22 mbar bei 58 bis 59°C. Das $^{19}$F-NMR-Spektrum zeigte ein charakteristisches Signal bei $\delta = -0,1$ ppm (gemessen mit $CF_3CO_2H$ als externem Standard).

### Beispiel 3

Zu 280 g (2,0 Mol) Phosphorpentoxid wurden 836 g eines Gemisches der Zusammensetzung 86 g Trifluormethansulfonsäure (erhalten nach Beispiel 2) und 750 g 86%iger Schwefelsäure gegeben. Danach wurde aus dem Gemisch wasserfreie Trifluormethansulfonsäure destilliert. Bei einem Druck von 18 mbar wurden bei 63 bis 67°C 44 g wasserfreie Trifluormethansulfonsäure isoliert. Das entspricht 51% der Theorie, bezogen auf umgesetztes $CF_3SSCF_3$.

### Beispiel 4

Zu einer Lösung von 200 g (1,0 Mol) $CF_3SSCF_3$ und 1000 g (12,5 Mol) $SO_3$ in 1437 g 96 gew.-%iger Schwefelsäure wurden unter Eiskühlung 280 g (4,1 Mol) 50%iges wäßriges Wasserstoffperoxid hinzugefügt. Die Reaktionsmischung wurde bei 20°C 2 Stunden nachgerührt und anschließend durch Destillation die flüchtigen Bestandteile abgetrennt. Die Redestillation der so abgetrennten flüchtigen Bestandteile lieferte 52 g (0,35 Mol) wasserfreie Trifluormethansulfonsäure mit einem Siedepunkt von 59 bis 63°C bei 20 mbar. Das entspricht einer Ausbeute von 26% der Theorie bezogen auf Wasserstoffperoxid.

## Ansprüche

1. Verfahren zur Herstellung von Trifluormethansulfonsäure, dadurch gekennzeichnet, daß man Bis-(trifluormethyl)-disulfan in flüssiger Phase, bei –20 bis + 100°C und in einem sauren Milieu mit einem sauerstoffhaltigen Oxidationsmittel behandelt.

2. Verfahren nach Anspruch 1, dadurch gekennzeichnet, daß man in Gegenwart einer starken Säure arbeitet, die bei den Reaktionsbedingungen flüssig ist.

3. Verfahren nach Anspruch 2, dadurch gekennzeichnet, daß die Säure wasserfrei oder wasserarm ist.

4. Verfahren nach Ansprüchen 2 bis 3, dadurch gekennzeichnet, daß man pro Gewichtsteil Bis-(trifluormethyl)-disulfan 5 bis 20 Gewichtsteile Säure einsetzt.

5. Verfahren nach Ansprüchen 1 bis 4, dadurch gekennzeichnet, daß man als Oxidationsmittel peroxidischen Sauerstoff enthaltende Stoffe, anorganische Persäuren, Salze anorganischer Persäuren, organische Persäuren, Peroxosalze, mit Fluor substituierte Schwefel-Peroxoverbindungen, aktiven Sauerstoff enthaltende Verbindungen, Salpetersäure, Nitrate, Oxide von Metallen in höheren Wertigkeitsstufen, entsprechende Säuren, entsprechende Salze und/oder molekularen Sauerstoff einsetzt.

6. Verfahren nach Anspruch 5, dadurch gekennzeichnet, daß man als Oxidationsmittel Wasserstoffperoxid, Peressigsäure, Perpropionsäure, Perbenzoesäure, 3-Chlor-perbenzoesäure, Peroxosulfate, Peroxodisulfate oder Caro'sche Säure einsetzt.

7. Verfahren nach Ansprüchen 1 bis 6, dadurch gekennzeichnet, daß man 2 bis 20 Äquivalente Oxidationsmittel pro Mol Bis-(trifluormethyl)-disulfan einsetzt.

8. Verfahren nach Ansprüchen 1 bis 7, dadurch gekennzeichnet, daß man bei Drucken von 1 bis 6 bar arbeitet.

9. Verfahren nach Ansprüchen 1 bis 8, dadurch gekennzeichnet, daß man aus dem nach der Behandlung mit dem sauerstoffhaltigen Oxidationsmittel vorliegenden Reaktionsgemisch Trifluormethansulfonsäure durch Destillation gewinnt.

## Claims

1. Process for the preparation of trifluoromethanesulphonic acid, characterized in that bis-(trifluoromethyl) disulphane is treated in the liquid phase, at – 20 to + 100°C and in an acidic medium with an oxygen-containing oxidizing agent.

2. Process according to Claim 1, characterized in that the reaction is carried out in the presence of a strong acid which is liquid under the reaction conditions.

3. Process according to Claim 2, characterized in that the acid is anhydrous or low in water.

4. Process according to Claims 2 to 3, characterized in that 5 to 20 parts by weight of acid are employed per part by weight of bis-(trifluoromethyl) disulphane.

5. Process according to Claims 1 to 4, characterized in that peroxidic oxygen-containing substances, inorganic peracids, salts of inorganic peracids, organic peracids, peroxo salts, sulphur-peroxo compounds substituted with fluorine, active oxygen-containing compounds, nitric acid, nitrates, oxides of metals in higher valency states, corresponding acids, corresponding salts and/or molecular oxygen are employed as oxidizing agents.

6. Process according to Claim 5, characterized in that hydrogen peroxide, peracetic acid, perpropionic acid, perbenzoic acid, 3-chloro-perbenzoic acid, peroxosulphates, peroxodisulphates or Caro's acid are employed as oxidizing agents.

7. Process according to Claims 1 to 6, characterized in that 2 to 20 equivalents of oxidizing agent are employed per mol of bis-(trifluoromethyl) disulphane.

8. Process according to Claims 1 to 7, characterized in that the reaction is carried out at pressures of 1 to 6 bar.

9. Process according to Claims 1 to 8, characterized in that trifluoromethanesulphonic acid is recovered by distillation from the reaction mixture present after the treatment with the oxygen-containing oxidizing agent.

## Revendications

1. Procédé de production d'acide trifluorométhanesulfonique, caractérisé en ce qu'on traite du bis(trifluorométhyl)disulfane en phase liquide entre – 20 et + 100°C et en milieu acide avec un agent oxydant contenant de l'oxygène.

2. Procédé suivant la revendication 1, caractérisé en ce qu'on opère en présence d'un acide fort qui est liquide dans les conditions de la réaction.

3. Procédé suivant la revendication 2, caractérisé en ce que l'acide est anhydre ou contient peu d'eau.

4. Procédé suivant les revendications 2 et 3, caractérisé en ce qu'on utilise 5 à 20 parties en poids d'acide par partie en poids de bis(trifluorométhyl)-disulfane.

5. Procédé suivant les revendications 1 à 4, caractérisé en ce qu'on utilise comme oxydants des substances peroxydiques contenant de l'oxygène, des peracides inorganiques, des sels de peracides inorganiques, des peracides organiques, des peroxosels, des peroxo-composés contenant du soufre substitués avec du fluor, des composés contenant de l'oxygène actif, l'acide nitrique, des nitrates, des oxydes de métaux à des degrés élevés de valence, des acides correspondants, des sels correspondants et/ou l'oxygène moléculaire.

6. Procédé suivant la revendication 5, caractérisé en ce qu'on utilise comme agent oxydant le peroxyde d'hydrogène, l'acide peracétique, l'acide perpropionique, l'acide perbenzoïque, l'acide 3-chloroperbenzoïque, des peroxosulfates, des peroxodisulfates ou l'acide de Caro.

7. Procédé suivant les revendications 1 à 6, caractérisé en ce qu'on utilise 2 à 20 équivalents d'agent oxydant par mole de bis(trifluorométhyl)disulfane.

8. Procédé suivant les revendications 1 à 7, caractérisé en ce qu'on opère à des pressions de 1 à 6 bars.

9. Procédé suivant les revendications 1 à 8, caractérisé en ce qu'on obtient l'acide trifluorométhanesulfonique par distillation à partir du mélange réactionnel obtenu après traitement avec l'agent oxydant contenant de l'oxygène.